# EUROPEAN PATENT SPECIFICATION

(11) **EP 4 262 567 B1**
(45) Date of publication and mention of the grant of the patent: **20.03.2024**
(21) Application number: 21820636.5
(22) Date of filing: 08.12.2021
(51) Int. Cl.: A61B 8/08, A61B 8/12, A61B 8/00

(54) **SYSTEM AND METHOD FOR DETERMINING POSITION INFORMATION**
SYSTEM UND VERFAHREN ZUR BESTIMMUNG VON POSITIONSINFORMATIONEN
SYSTÈME ET PROCÉDÉ PERMETTANT DE DÉTERMINER DES INFORMATIONS DE POSITION

(30) Priority: 17.12.2020 US 202063126856 P; 12.01.2021 EP 21151067
(43) Date of publication of application: 25.10.2023
(73) Proprietor: Koninklijke Philips N.V., 5656 AG Eindhoven (NL)
(72) Inventor: BHARAT, Shyam, 5656 AG Eindhoven (NL); CHEN, Alvin, 5656 AG Eindhoven (NL); RAJU, Balasundar, Iyyavu, 5656 AG Eindhoven (NL); SUTTON, Jonathon, Thomas, 5656 AG Eindhoven (NL); TOPOREK, Grzegorz, Andrzej, 5656 AG Eindhoven (NL)
(74) Representative: Philips Intellectual Property & Standards
(86) International application number: PCT/EP2021/084719
(87) International publication number: WO 2022/128664

(56) References cited:
- WO-A1-98/11840
- US-A1- 2014 276 003
- US-A1- 2016 324 501

## Description

### FIELD OF THE INVENTION

The invention relates to the field of determining position information, and in particular to determining position information for a device in the body of a subject.

### BACKGROUND OF THE INVENTION

The use of transesophageal echocardiography (TEE) has become increasingly popular for monitoring cardiac function during surgery. TEE enables high-quality real-time visualization of the heart.

In order to obtain a suitable echocardiogram, a TEE probe must be positioned in the esophagus at a location sufficiently close to the heart. For example, to view the left ventricle, the TEE probe must be in the mid-esophageal position. Correctly positioning the probe requires dexterity and clinical expertise.

Various image-based solutions have been proposed to guide a non-expert user in positioning a TEE probe within the esophagus. These solutions rely on identifying features in ultrasound images obtained by the probe. However, in order to obtain ultrasound images, the transducer array elements of the probe must be in contact with tissue; this is not always the case when first inserting a TEE probe. Further, not all probe positions provide images with features that can be used to identify the position. This is particularly the case for the upper section of the esophagus.

There is therefore a need for an improved mechanism for allowing a non-expert user to position a TEE probe.

US 2016/324501 A1 describes a tool navigation system that track a position of an interventional tool relative to an acoustic image plane using ultrasound signals.

### SUMMARY OF THE INVENTION

The invention is defined by the claims.

According to examples in accordance with an aspect of the invention, there is provided a processing system for determining position information for a device within a body of a subject, wherein the device is capable of outputting and/or routing an acoustic signal having a frequency of no more than 20 kHz.

The processing system is configured to: receive, from a first sensor positioned externally to the body of the subject, a first received signal responsive to the acoustic signal output and/or routed by the device; obtain a value for one or more parameters of the first received signal, wherein each of the one or more parameters is a parameter of the first received signal that varies with the distance traveled by the acoustic signal; and process the value for each of the one or more parameters to determine position information for the device.

This system may be used to obtain position information about a device inside the body in situations where image-based position information cannot be practicably obtained or is inconclusive. The position information may, for instance, define a relative position of the device within the body, with respect to the position of the first sensor.

The inventors have recognized that many devices inserted in a subject for imaging the subject are capable of transmitting acoustic signals (having an audio/audible frequency) in some operating modes, and that an acoustic signal transmitted by the device inside the body may be received by an external sensor even when the position of the device is such that imaging data cannot be generated. In particular, standard (i.e. non-specialized) equipment usable for detecting audible sounds, such as stethoscopes, can be used, meaning that position information of the device can be obtained or determined without the need for dedicated or adapted technology. An audio frequency is a frequency within the range of human hearing, i.e. no more than 20 kHz, e.g. from 20 Hz to 20 kHz inclusively. Thus, the device may be capable of outputting an acoustic signal having a frequency of no more than 20 kHz, e.g. from 20 Hz to 20 kHz, and the first sensor may be similarly adapted to be responsive to acoustic signals in this frequency range.

A further advantage of using audio frequency signals (less than 20 kHz) is that such audio signals have improved penetration through material than higher frequency signals (e.g. ultrasound signals).

A number of parameters of received acoustic signals vary in value depending on the distance traveled by the signal between transmission and reception. For example, the intensity of an acoustic signal decreases as the distance traveled by the signal increases. Such parameters may therefore be used to determine position information for the device.

The determined position information may comprise a position of the device relative to the sensor and/or a position of the device relative to a former or reference position of the device.

In some embodiments, the device is configured to emit acoustic signals having a frequency of no more than 20 kHz during an ultrasound imaging process. Thus, the acoustic signals (of a frequency <=20 kHz) are produced as a side-effect of the ultrasound imaging process performed inside the subject, meaning that previously unused signals are employed to track or identify the position of the device. This approach also means that a second ultrasound detector (e.g. to be positioned external) is not required, rather a readily available and cheap audible sensor can be used instead, increasing a flexibility of use.

In some ultrasound imaging modes, the imaging array elements of an ultrasound imaging device that will emit audible sounds alongside the ultrasound transmit pulses. For example, sound is emitted in Color Doppler mode due to the long pulse lengths of the Doppler pulses in Color Doppler mode.

This embodiment thereby makes use of an unintended emission of audible sound during ultrasound imaging to track and identify a position of an ultrasound imaging device during the probe insertion process. This can facilitate the use of standard equipment, e.g. standard stethoscopes or the like, to detect the position of an ultrasound imaging probe.

In some embodiments, the device comprises a transesophageal echocardiography probe. The first sensor may be, while in use, positioned on the thorax of the subject.

The one or more parameters of the first received signal may comprise at least one of: an intensity of the first received signal, an amplitude of the first received signal, a time-of-flight of the first received signal and/or a frequency of the first received signal.
The intensity and amplitude of a received signal decrease as the distance traveled between transmission and reception increases.

The time-of-flight of a received signal increases as the distance traveled between transmission and reception increases.

The frequency of a received signal is Doppler shifted if the device is moving with respect to the sensor. The frequency of the received signal will be increased with respect to the frequency of the transmitted signal if the device is moving towards the sensor and decreased if the device is moving away from the sensor. If the path traveled by the acoustic signal between the device and the sensor is at a non-zero angle with respect to the path of movement of the device, the amount by which the frequency is shifted increases with distance.

In some embodiments, the processing system is further configured to repeat the steps of receiving a received signal and obtaining a value for one or more parameters of the received signal for a plurality of positions of the device; and the step of processing the value for each of the one or more parameters to determine position information for the device comprises processing the values corresponding to each of the plurality of positions of the device to determine a position of the device corresponding to at least one of: a maximum value of a parameter, a minimum value of a parameter, and/or a value of a parameter corresponding to an inflection point.

Parameters that increase in value as the distance traveled by the acoustic signal increases, such as time-of-flight, will have a minimum value at a position closest to the sensor. Parameters that decrease in value as the distance traveled by the acoustic signal increases, such as amplitude or intensity, will have a maximum value at a position closest to the sensor. Parameters that vary according to whether a moving device is moving towards or away from the sensor, such as frequency, will have an inflection point at a position closest to the sensor.

These values may therefore be used to identify which of the plurality of positions of the device is the closest to the sensor. The sensor may be positioned such that a closest position of the device corresponds to a desired position of the device. For example, when the device is a transesophageal echocardiography (TEE) probe, a desired position may be the mid-esophageal position, which is closest to the heart and a suitable position for imaging the cardiac anatomy. By positioning a sensor on the subject's chest at a position close to the heart, the position of a TEE probe closest to the sensor is the mid-esophageal position.

In some embodiments, the step of processing the value for each of the one or more parameters to determine position information for the device further uses a reference value for each of the one or more parameters corresponding to a reference position of the device; and the position information comprises a position of the device relative to a reference position.

The reference position may be a desired or known position of the device achieved by a positioning of the device by a clinical expert. Determining the position of the device relative to the reference position may be used to aid a user in repositioning the device correctly.

In some embodiments, the processing system is further configured to: receive, from a second sensor positioned externally to the body of the subject in a different position to the first sensor, a second received signal responsive to the same acoustic signal output and/or routed by the device; and obtain a value for one or more parameters of the second received signal, wherein each of the one or more parameters corresponds to a parameter of the first received signal (e.g. is the same type of parameter); and the step of processing the value for each of the one or more parameters to determine position information for the device comprises processing the values for the first and second received signals to determine a position of the device relative to each of the first and second sensors.

If more than one sensor is used to receive the acoustic signal transmitted by the device, the received signals may be used to determine the position of the device with respect to the sensors. Since the sensors are, when in use, positioned externally to the subject's body, their positions are known; the position of the device relative to the sensors may therefore be used to determine the position of the device within the subject's body. For instance, the position of the device can be identified using a multilateration and/or triangulation approach.

For example, the position of the device with respect to each sensor may be determined by processing the time-of-flight of the acoustic signal received at each sensor.

In some embodiments, the processing system is further configured to repeat the steps of receiving a received signal and obtaining a value for one or more parameters of the received signal for a plurality of different acoustic signals output and/or routed by the device from a single position; and the step of processing the value for each of the one or more parameters to determine position information for the device comprises processing the values for each of the received signals.

Different acoustic signals may, for example, have different frequencies. If the device is an ultrasound imaging device, different acoustic signals may be produced using different imaging modes.

Determining the position information using values obtained from multiple acoustic signals transmitted from a single position improves the reliability of the determined position information.

The different acoustic signals transmitted by the device may be chosen according to the type of sensor used to receive the signals. For example, if the sensor is a stethoscope, the device may be configured to transmit a high pitched signal, which would be detected by the diaphragm of the stethoscope, and a low pitch signal, which would be detected by the bell of the stethoscope.

In some embodiments, the processing system is further configured to: receive information from the device responsive to the one or more acoustic signals output and/or routed by the device; and determine whether a received signal corresponds to one of the one or more acoustic signals output and/or routed by the device.

In this way, signals received by the sensor that do not correspond to a signal transmitted by the device may be disregarded. This would reduce the effect of false positives on the determined position information.

The information corresponding to a transmitted signal may, for example, comprise a time of transmission.

In some embodiments, the processing system is further configured to output an indication of the determined position information to a display device.

An indication of the determined position information may comprise an indication that the device is in a desired position, an indication of the distance between the device and a desired position, and/or an indication of the direction in which the device must be moved to reach a desired position. The display device may be configured to provide a visual representation of the indication of the determined position information, e.g. in the form of a textual display, a visual display and so on.

In some embodiments, the device is an ultrasound imaging device; the step of processing the value for each of the one or more parameters to determine position information for the device comprises determining whether the device is in a desired position; and the processing system is further configured to instruct a display device to display an image corresponding to imaging data received by the device in response to determining that the device is in a desired position.

In this way, a user may decide whether the position of the device achieves a suitable imaging plane based on the displayed image and make further adjustments to the position of the device if required.

This combination of audio-based position information and image-based position information may allow the device to be positioned with a greater accuracy than either one of audio-based position information and image-based position information alone.

There is also proposed a system for determining position information for a device within a body of a subject, wherein the device is capable of outputting and/or routing an acoustic signal having a frequency of no more than 20 kHz. The system comprises: at least one sensor positioned, when in use, externally to the body of the subject and configured to receive an acoustic signal output and/or routed by the device; and the processing system described above.

According to another aspect of the invention, there is provided a computer-implemented method for determining position information for a device within a body of a subject, wherein the device is capable of outputting and/or routing an acoustic signal having a frequency of no more than 20 kHz.

The computer-implemented method comprises: receiving, from a first sensor positioned, when in use, externally to the body of the subject, a first received signal responsive to the acoustic signal output and/or routed by the device; obtaining a value for one or more parameters of the first received signal, wherein each of the one or more parameters is a parameter of the first received signal that varies with the distance traveled by the acoustic signal; and processing the value for each of the one or more parameters to determine position information for the device.

There is also proposed a computer program product comprising computer program code means which, when executed on a computing device have a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for determining position information for a device within a body of a subject, according to an embodiment of the invention;
Fig. 2 shows a graph illustrating the values obtained for the amplitude of received signals received by a sensor as a device is moved down a subject's esophagus, in accordance with an aspect of the invention;
Fig. 3 shows a graph illustrating the values obtained for the frequency of received signals received by a sensor as a device is moved down a subject's esophagus, in accordance with an aspect of the invention;
Fig. 4 illustrates a method for positioning a device within a subject's body, according to an embodiment of the invention;
Fig. 5 illustrates a system for determining position information for a device within a body of a subject, according to an embodiment of the invention; and
Fig. 6 illustrates a computer-implemented method for determining position information for a device within a body of a subject, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and method for determining position information for a device within a body of a subject. The device outputs and/or routes an acoustic signal with a frequency of no more than 20 kHz, which is received by at least a first sensor, positioned externally to the body of the subject. A processing system receives the received signal from the sensor, obtains a value for one or more parameters of the received signal and processes the value(s) to determine position information for the device.

Embodiments are at least partly based on the realizations that some devices inserted into a subject's body during a clinical procedure naturally emit audible-frequency sound in some operating modes, that audible sound emitted inside the body may be detected by an external sensor, and that certain parameters of this audible sound may be used as a marker of the position of the device.

Illustrative embodiments may, for example, be employed in ultrasound systems, such as ultrasound-based cardiac monitoring systems, laparoscopic ultrasound systems, and intravascular ultrasound systems.

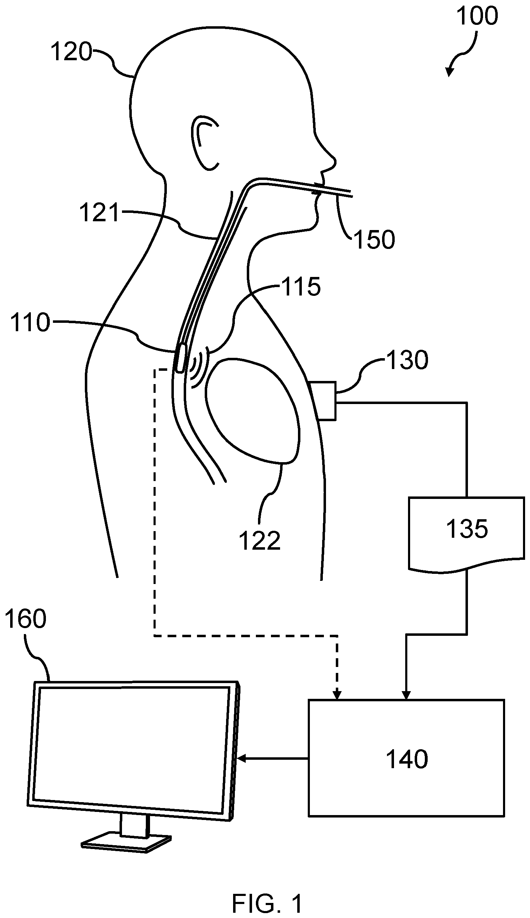

Fig. 1 illustrates a system 100 for determining position information for a device 110 within a body of a subject 120, according to an embodiment of the invention. The system comprises a sensor 130 and a processing system 140. The processing system 140 is, itself, an embodiment of the invention.

The device 110 shown in Fig. 1 is a transesophageal echocardiography (TEE) probe; however, the system 100 may be used to determine position information for any suitable device in the body. A suitable device is any device designed to be positioned in a subject's body and capable of outputting and/or routing an acoustic signal with a frequency lower than ultrasound frequencies, that is, a frequency of no more than 20 kHz - i.e. output and/or route an "audible signal". For example, the device may be a device for interventional ultrasound, such as a laparoscopic ultrasound probe or an intravascular ultrasound probe. Alternatively, the device may be a device for an interventional procedure that does not typically generate an acoustic signal, and an acoustic signal may be generated intentionally for tracking purposes.

In Fig. 1, the device 110 is at a distal end of a gastroscope 150, and has been inserted into the esophagus 121 of the subject 120. Fig. 1 shows the device 110 in a mid-esophageal position, which is close to the subject's heart 122 and is a suitable position for obtaining images of the heart.

The TEE probe 110 emits an audible signal/sound (i.e. having a frequency within the range of human hearing) in some imaging modes, such as Color Doppler mode. The sound is emitted by the imaging array elements in the TEE probe due to the long pulse lengths of the Doppler pulses in Color Doppler mode. The frequency of this sound is proportional to the pulse repetition frequency of the imaging transmit pulses.

The device 110 may therefore emit an audible signal by operating in an imaging mode that happens (e.g. but not as its principle/primary purpose) to emit audible signals. The imaging data obtained while the device is operating in this imaging mode may or may not be used to obtain images. In another example, the device may operate in a customized mode configured so that the device emits an acoustic signal with the desired characteristics (e.g. a frequency between 20 Hz and 20 kHz).

In other examples, other types of devices 110 may also emit an audible signal/sound as a side-effect of operation, or they may be intentionally configured to emit an audible signal/sound, for example, by being fitted with an additional component capable of producing audible signal/sound. Alternatively, the sound may not be generated within the device; rather, the audible signal/sound may be produced elsewhere (e.g. externally to the gastroscope 150) and directed/routed by the device. The audible signal/sound may be generated in one part of the device, and directed by another part of the device in a desired direction.

An audible signal 115 emitted and/or routed by the device 110 is received by the sensor 130, which is positioned externally to the body of the subject 120. In Fig. 1, the sensor 130 is placed on the thorax of the subject, close to the subject's heart 122. The sensor 130 may be any sensor capable of receiving a signal having a frequency of no more than 20 kHz. For example, the sensor 130 may be a stethoscope, or an ultrasound probe fitted with a microphone tuned to the frequency range of the sound emitted and/or routed by the device. If an ultrasound probe fitted with a microphone is used, the probe may also be used to detect ultrasound signals from the device 110 in order to obtain additional data that may be used in determining position information.

The sensor 130 sends a received signal 135, responsive to the acoustic signal 115, to the processing system 140. The processing system 140 obtains a value for one or more parameters of the received signal. The one or more parameters of the received signal comprise one or more parameters that vary depending on the distance traveled by the acoustic signal 115. Suitable parameters will be apparent to the skilled person, and may, for example, include amplitude, intensity, time-of-flight, and, in the case of a signal from a moving device, frequency.

The processing system 140 processes the obtained value(s) for the one or more parameters to determine position information for the device 110. Various methods for determining position information based on values for one or more parameters of the received signal 135 are envisioned, and are described below.

In an example, the device 110 is inserted into the subject's mouth and moved down the esophagus 121 by the use of the gastroscope 150, as shown in Fig. 1. Audible signals 115 (i.e. acoustic signals having a frequency less than 20 kHz or between 20 Hz and 20 kHz) are emitted and/or routed as the device is moved, and each signal is received by the sensor 130. In other words, an acoustic signal is emitted and/or routed by the device at each of a plurality of positions along the esophagus. The device 110 may output an acoustic signal continuously as the device is moved down the esophagus.

The processing system 140 receives the received signals 135 from the sensor 130 and obtains a value for one or more parameters for each received signal, thus obtaining a plurality of values, corresponding to the plurality of positions of the device 110, for each of the one or more parameters.

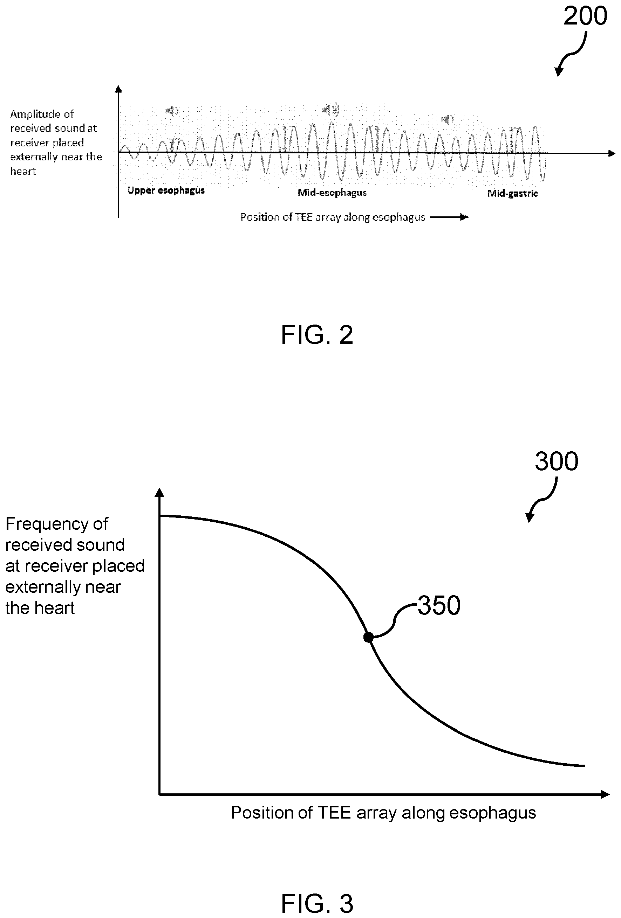

Fig. 2 shows a graph 200 illustrating the values obtained for the amplitude of received signals 135 received by the sensor 130, positioned as shown in Fig. 1, as the device 110 is moved down the esophagus 121.

As the graph 200 shows, the amplitude of signals received by the sensor increases as the distance between the device and the sensor decreases. The smaller the distance traveled by the acoustic signal 115, the less energy is absorbed by the surroundings before the signal is received at the sensor, so the greater the amplitude of the received signal.

The graph 200 shows that the amplitude of the received signal 135 reaches a maximum when the device 110 is in a mid-esophageal position. The position of the external sensor 130 is arranged so that the device 110 is closest to the sensor at a mid-esophageal position. Due to the shape of the esophagus, the device moves away from the sensor as the device moves down away from the mid-esophageal position, causing the amplitude of received signals to decrease, and then begins to move towards the sensor again as the device approaches a mid-gastric position, causing the amplitude of received signals to increase again.

The device 110 can therefore be positioned in a desired mid-esophageal position by moving the device down the esophagus until a first maximum amplitude is achieved in the amplitude values for received signals responsive to acoustic signals output and/or routed by the device. The device may be moved beyond the position corresponding to the first maximum amplitude in order to identify the first maximum amplitude, and then moved back to the position corresponding to the first maximum amplitude.

Fig. 3 shows a graph 300 illustrating the values obtained for the frequency of received signals 135 received by the sensor 130, positioned as shown in Fig. 1, as the device 110 is moved down the esophagus 121.

Due to the Doppler effect, the frequency of the received signal 135 depends on the movement of the device 110 relative to the sensor 130. As the device is moved towards the sensor, the frequency of the signal received at the sensor is increased relative to the frequency of the signal at emission. Since the device is not moving directly towards the sensor, the amount by which the frequency is increased decreases as the distance between the device and the sensor decreases. As the device is moved away from the sensor, the frequency of the received signal is decreased relative to the frequency of the emitted signal.

The inflection point 350 on the graph 300 corresponds to the point at which the movement of the device changes from moving towards the device to moving away from the device. In other words, the inflection point 350 corresponds to the position at which the device moves past the sensor. The sensor may be positioned so that this position is the mid-esophageal position, although other suitable positions for the sensor to achieved particular positions for the device will be apparent from this teaching.

Similarly, the processing system 140 may identify when the device is in the mid-esophageal position by identifying the position at which an intensity of received signals 135 reaches a maximum, or a time-of-flight reaches a minimum, as the device 110 is moved from an upper esophageal position down the esophagus 121.

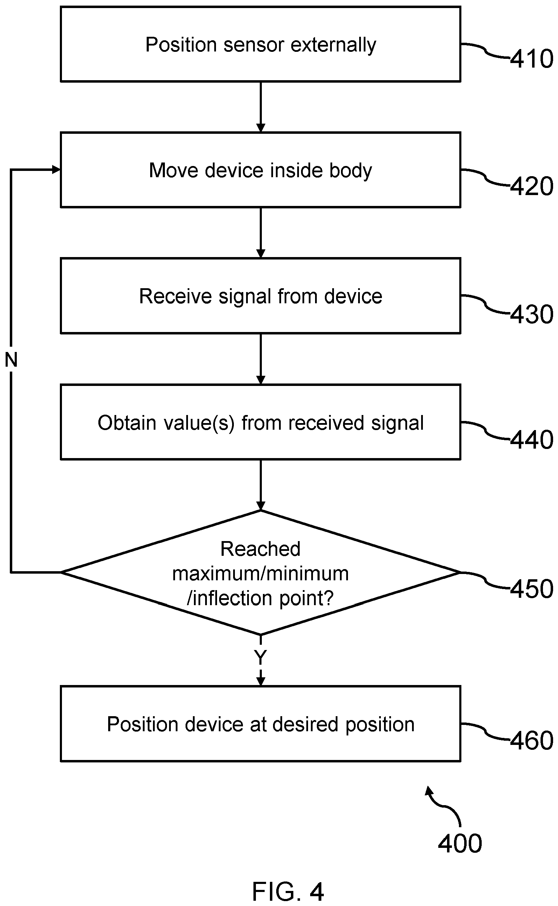

Fig. 4 illustrates a method 400 for positioning a device within a subject's body, according to an embodiment of the invention. The method may be used to position any device capable of outputting and/or routing an acoustic signal having a frequency of no more than 20 kHz. For example, the device may be the device 110 of Fig. 1.

The method begins with step 410, in which a sensor is positioned externally to the subject's body. The sensor is any sensor capable of receiving an acoustic signal with a frequency of no more than 20 kHz (i.e. an "audible signal"). For example, the sensor may be the sensor 130 of Fig. 1. The sensor may be positioned according to a desired position of the device within the subject's body, in order that a parameter of an acoustic signal from the device and received by the sensor has a maximum value, a minimum value, or a value corresponding to an inflection point when the device is at the desired position. For example, the sensor may be positioned such that the device is closer to the sensor when the device is at the desired position than at any other position along the device's path of movement.

At step 420, the device is moved inside the subject's body. For example, the device may be moved down the subject's esophagus, as shown in Fig. 1. The device outputs and/or routes an acoustic signal as it is moved. For example, in the case of an ultrasound device, the device may output an acoustic signal by operating in a mode in which audio pulses are emitted, such as Color Doppler mode.

At step 430, the acoustic signal is received by the sensor. A processing system receives the received signal from the sensor.

At step 440, the processing system obtains a value for one or more parameters of the received signal. The one or more parameters vary with the distance traveled by the acoustic signal, as described above. For example, the processing system may obtain a value for one or more of: an intensity of the received signal, an amplitude of the received signal, a time-of-flight of the received signal and a frequency of the received signal.

At step 450, the processing system determines whether the value for each parameter has reached one of: a maximum value, a minimum value or a value corresponding to an inflection point. Which of these the processing system determines depends on the how parameter varies with distance traveled. For example, the processing system may determine whether the value of the amplitude of the received signal has reached a maximum, as described with reference to Fig. 2 above.

The position of the external sensor and the path of movement of the device may be such that there is more than one maximum/minimum/inflection point in each of the one or more parameters. The processing system may therefore determine whether the maximum value, minimum value or value corresponding to an inflection point corresponds to the desired position of the device.

For example, Fig. 2 shows that the amplitude of signals received at a sensor close to the subject's heart reaches a first maximum at the mid-esophageal position as the device moves down the subject's esophagus, and a second maximum further down the esophagus at the mid-gastric position. The processing system may therefore determine that the device is in the mid-esophageal position when the processing system determines that the device is at a position corresponding to a first maximum amplitude. In other examples, the processing system may identify the desired position of the device by determining that a second or later maximum, minimum or inflection point has been reached.

Returning to Fig. 4, if at step 450 the processing system determines that the value for each parameter has not reached a maximum value, a minimum value or an inflection point, or has not reached the maximum, minimum or inflection point corresponding to the desired position if this is not the first or only maximum, minimum or inflection point, the device continues to be moved further inside the subject's body, and the steps of receiving an acoustic signal from the device and obtaining a value for one or more parameters of the received signal are repeated.

Steps 420 to 450 continue to be repeated until the processing system determines that the value for each of the one or more parameter has reached a maximum value, a minimum value or an inflection point, or has reached the maximum, minimum or inflection point corresponding to the desired position if this is not the first or only maximum, minimum or inflection point.

At step 460, the device is positioned at the desired position within the subject's body. If the processing system determines that a maximum, minimum or inflection point in a parameter has been reached while the device is at the position corresponding to the maximum, minimum or inflection point, the movement of the device can be halted so that the device remains at the desired point. The processing system may, for example, be able to identify a maximum, minimum or inflection point while the device is at the position corresponding to the maximum, minimum or inflection point if the method 400 has previously been used to position the device within the subject, by comparing the obtained value(s) with a value previously identified as corresponding to a maximum, minimum or inflection point.

The processing system may identify a maximum, minimum or inflection point only once the device has been moved past the maximum, minimum or inflection point. For example, the processing system may determine that a value corresponds to a maximum, minimum or inflection point based on values from positions either side of the position corresponding to the value. The desired position may therefore be achieved by moving the device back to the position corresponding to the determined maximum, minimum or inflection point.

Returning to Fig. 1, in another example, the device 110 may be initially positioned in a desired position, such as the mid-esophageal position, by a clinical expert. The sensor 130 receives an acoustic signal output and/or routed by the device while it is in its initial position, and sends the received signal to the processing system 140.

The processing system 140 obtains a value for one or more parameters of the received signal. In this way, the processing system obtains one or more reference values corresponding to a signal from the desired position.

The device 110 is then moved by a user, who may not be a clinical expert. The sensor 130 receives an acoustic signal output and/or routed by the device from its new position, and sends the received signal to the processing system 140. The processing system obtains a value for one or more parameters of the received signal. At least one of the one or more parameters should correspond to one of the parameters for which a reference value has been achieved.

The processing system 140 compares the value(s) corresponding to the new position with the reference value(s) in order to determine a relative position of the device 110. For example, the processing system may determine whether or not the position of the device is the same as the initial position. In another example, a calibration process may be used so that the new position of the device relative to the initial position may be determined. Ground truth knowledge of the distance moved by the device may be obtained, for example, using electromagnetic tracking, distance markers on the device, and so on, and the processing system may use the value(s) corresponding to the new position, the reference value(s) and the ground truth value of the distance moved to determine the relative position of the device.

The processing system 140 may determine how the device 110 should be moved in order to return the device to the initial position. For example, a decrease in frequency relative to the reference frequency would indicate that the device is moving away from the sensor 130. If the sensor 130 is positioned such that the initial position of the device is the closest position to the sensor, the processing system would determine that the device should be moved in the opposite direction to a current direction of movement in order to move towards the initial position.

In some clinical procedures, it may be advantageous to move the device 110 between a plurality of desired positions. A clinical expert may position the device in each of the plurality of desired positions, and a value for one or more parameters of a received signal corresponding to a signal from each of the plurality of positions may be obtained. A non-expert user may then navigate the device between the desired positions as required by the processing system comparing the value(s) for a received signal corresponding to a current position to the value(s) from each of the desired positions.

In some embodiments, the system 100 may be configured such that acoustic signals that do not correspond to signals emitted and/or routed by the device 110 are disregarded. For example, the device and sensor 130 may be connected, and the sensor may be configured to be active only within a predefined time period from a time at which a signal has been output and/or routed by the device. The predefined time period may, for example, be determined based on an expected maximum time-of-flight of the acoustic signal.

The device 110 may be in communication with the processing system 140. For example, the processing system may be configured to receive information responsive to acoustic signals output and/or routed by the device and determine whether a received signal corresponds to an acoustic signal output and/or routed by the device. The information responsive to a signal output/routed by the device may comprise any characteristic(s) of the signal that may be used to distinguish the signal from other signals received by the sensor 130. For example, the information may comprise a time of outputting/routing, and the processing system may determine whether a received signal corresponds to the output/routed signal based on the time of outputting/routing, a time of reception and an expected time-of-flight.

In some embodiments, the device 110 may output and/or route a plurality of different acoustic signals from one position, and the processing system 140 may obtain a value for one or more parameters for a plurality of received signals corresponding to the plurality of output/routed signals.

Methods for configuring the device 110 to output and/or route a plurality of different acoustic signals will be apparent to the skilled person. For example, if the device is an ultrasound device, the device may be configured to emit different acoustic signals (e.g. signals with different frequencies) by switching between different operating modes (e.g. 2D, 3D, Color Doppler etc.), or by operating in a customized mode in which the device emits signals of varying characteristics (e.g. amplitude, frequency, etc.).

The acoustic signal(s) output and/or routed by the device 110 may be tailored to the sensor 130. For example, if the sensor is a stethoscope, the device may be configured to output and/or route alternating high and low pitch signals. The diaphragm of the sensor would receive the high pitch signals, and the bell of the stethoscope the low pitch signals, resulting in a higher SNR than a uniform signal. A high pitch signal may have a frequency in the range of 100-500 Hz and a low pitch signal may have a frequency in the range of 20-100 Hz.

Different acoustic signals from a single position of the device 110 may be used to improve the reliability of the determined position information for embodiments in which signals are output and/or routed from a plurality of positions. The same set of different acoustic signals may be output and/or routed from different positions of the device 110, and the processing system 140 may thus obtain a set of values for each of the one or more parameters corresponding to each position.

For example, the processing system may compare a set of values for each parameter corresponding to a current position with a corresponding set of reference values to more reliably determine a position of the device relative to a reference position.

In some embodiments, the system 100 further comprises a display device 160. The processing system 140 may be configured to output an indication of the determined position information to the display device. The displayed indication may, for example, comprise text and/or an image.

The displayed indication depends on the type of position information determined by the processing system 140, and may comprise, for example, at least one of: an indication that a current position of the device 110 corresponds to a maximum value of a parameter of the received signal, a minimum value of a parameter of the received signal, or a value of a parameter of the received signal corresponding to an inflection point; an indication of a required direction of movement of the device in order to position the device at a position corresponding to a maximum value of a parameter of the received signal, a minimum value of a parameter of the received signal, or a value of a parameter of the received signal corresponding to an inflection point; a position of the device relative to a reference position; an indication that a current position of the device corresponds to a reference position; an indication of a required direction of movement of the device in order to position the device at a reference position; and/or an indication of a distance between a current position of the device and a desired position of the device.

In some embodiments, the device 110 is an imaging device, such as an ultrasound imaging device, and the processing system 140 is configured to determine whether the device is in a desired position. The processing system 140 may be further configured to cause a display on the display device 160 to transition to an image based on imaging data from the device 110 if the processing system determines that the device is in a desired position. The transition to displaying an image may serve as an indication that the device is in the desired position.

Determining that the device 110 is in a desired position may comprise, for example, determining that the device is in a position corresponding to a maximum value of a parameter of the received signal, a minimum value of a parameter of the received signal, or a value of a parameter of the received signal corresponding to an inflection point, or that the device is in a reference position.

The device 110 may obtain imaging data while the device is being positioned, for example if the acoustic signals from the device are a side-effect of the operation of an ultrasound imaging mode, such as Color Doppler mode. If a current operation mode is suitable for the visualizing anatomy in the desired position, the operation of the device may continue in the same mode once the processing system detects that the device is in a desired position. Alternatively, the operation mode of the device may be changed to a more suitable mode for imaging the targeted anatomy. For example, in the case of transesophageal echocardiography, the operation mode of the device may be changed to B-mode in order to image the cardiac anatomy.

Displaying an image from the device 110 once the processing system 140 determines that the device is in the desired position allows an image-based guidance system to be used to finalize the position of the device. A user of the system may confirm that a current view corresponds to a desired view of the anatomy, and further adjust the position of the probe if the current view does not correspond to the desired view. Typically, when the device is in the vicinity of the desired position, images from the device will contain identifiable anatomical features that may be used to guide a user in repositioning a device.

Alternatively, the display device 160 may display one or more images based on imaging data from the device 110 throughout the positioning of the device. In this way, the audio-based positioning may be combined with image-based position guidance.

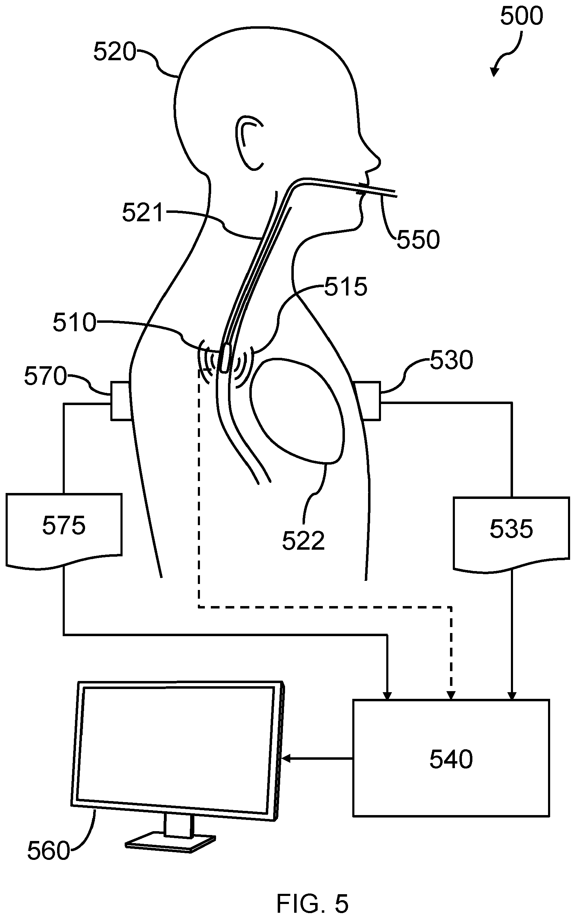

Fig. 5 illustrates a system 500 for determining position information for a device 510 within a body of a subject 520, according to another embodiment of the invention. The system 500 is similar to the system 100 illustrated in Fig. 1, but the system 500 comprises a first sensor 530 and a second sensor 570.

The use of multiple sensors 530, 570 allows the position of the device 510 to be identified using a triangulation and/or multilateration technique. In Fig. 5, two sensors are used, but further sensors may be used to improve the precision of the determined position information.

In Fig. 5, the second sensor 570 is positioned on the opposite side of the subject 520 to the first sensor 530, but the sensors may be placed in any positions capable of receiving acoustic signals from the device 510. For example, both sensors 530, 570 may be positioned on the anterior side of the subject, with one sensor on each side of the midline of the subject, or both sensors may be positioned on the posterior side of the subject.

The device 510, which may be the same as device 110 in Fig. 1, outputs and/or routes an acoustic signal 515, which is received by both the first sensor 530 and the second sensor 570. Processing system 540, which may be the same as processing system 140 in Fig. 1, receives the received signal from both sensors. In other words, the processing system receives a first received signal 535 from the first sensor and a second received signal 575 from the second sensor, where both the first and second received signals are responsive to the same output/routed signal from the device.

The processing system 540 obtains a value for one or more parameters of the first received signal 535 and a value for one or more corresponding parameters of the second received signal 575. The processing system processes the values to determine a position of the device 510 with respect to each of the first sensor 530 and the second sensor 570.

For example, the processing system 540 may analyze a time-of-flight of the acoustic signal between the device 510 and each of the first sensor 530 and the second sensor 570 in order to determine a position of the device relative to the sensors. Since the positions of the external sensors relative to the body of the subject 520 are known, the position of the device within the body may be determined from the position of the device relative to the sensors, e.g. using triangulation and/or multilateration techniques.

The system 500 may further be used to carry out any of the methods described above with reference to system 100 of Fig. 1. For example, the use of multiple sensors may improve the reliability of methods that involve comparing one or more values of a received signal with one or more reference values, since values from multiple received signals are available for the comparison.

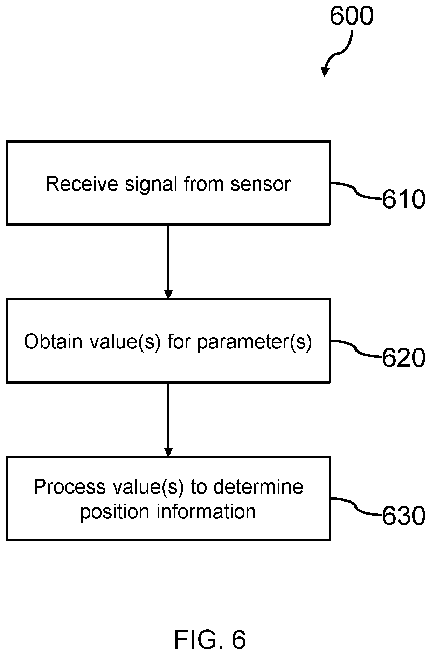

Fig. 6 illustrates a computer-implemented method 600 for determining position information for a device within a body of a subject, according to an embodiment of the invention. The method 600 may be used to determine position information for any device capable of outputting and/or routing an acoustic signal having a frequency of no more than 20 kHz.

The method begins with step 610, in which a first received signal responsive to the acoustic signal output and/or routed by the device is received from a first sensor positioned externally to the body of the subject.

At step 620, a value for one or more parameters of the first received signal is obtained, wherein each of the one or more parameters is a parameter of the first received signal that varies with the distance traveled by the acoustic signal.

At step 630, the value for each of the one or more parameters is processed to determine position information for the device.

It will be understood that the disclosed methods are computer-implemented methods. As such, there is also proposed a concept of a computer program comprising code means for implementing any described method when said program is run on a processing system.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing system (140, 540) for determining position information for a device (110, 510) within a body of a subject (120, 520), wherein the device is capable of outputting and/or routing an acoustic signal (115, 515) having a frequency of no more than 20 kHz, the processing system being configured to:
receive, from a first sensor (130, 530) positioned externally to the body of the subject, a first received signal (135, 535) responsive to the acoustic signal output and/or routed by the device;
obtain a value for one or more parameters of the first received signal, wherein each of the one or more parameters is a parameter of the first received signal that varies with the distance traveled by the acoustic signal; and
process the value for each of the one or more parameters to determine position information for the device.

2. The processing system (140, 540) of claim 1, wherein the device (110, 510) is configured to emit acoustic signals having a frequency of no more than 20 kHz during an ultrasound imaging process.

3. The processing system (140, 540) of claim 1 or 2, wherein the device (110, 510) comprises a transesophageal echocardiography probe.

4. The processing system (140, 540) of any of claims 1 to 3, wherein the first sensor (130, 530) is, when in use, positioned on the thorax of the subject (120, 520).

5. The processing system (140, 540) of any of claims 1 to 4, wherein the one or more parameters of the first received signal (135, 535) comprise at least one of: an intensity of the first received signal, an amplitude of the first received signal, a time-of-flight of the first received signal and/or a frequency of the first received signal.

6. The processing system (140, 540) of any of claims 1 to 5, wherein:
the processing system is further configured to repeat the steps of receiving a received signal and obtaining a value for one or more parameters of the received signal for a plurality of positions of the device (110, 510); and
the step of processing the value for each of the one or more parameters to determine position information for the device comprises processing the values corresponding to each of the plurality of positions of the device to determine a position of the device corresponding to at least one of: a maximum value of a parameter, a minimum value of a parameter, and/or a value of a parameter corresponding to an inflection point.

7. The processing system (140, 540) of any of claims 1 to 6, wherein:
the step of processing the value for each of the one or more parameters to determine position information for the device (110, 510) further uses a reference value for each of the one or more parameters corresponding to a reference position of the device; and
the position information comprises a position of the device relative to a reference position.

8. The processing system (140, 540) of any of claims 1 to 7, wherein:
the processing system is further configured to:
receive, from a second sensor (570) positioned externally to the body of the subject in a different position to the first sensor (130, 530), a second received signal (575) responsive to the same acoustic signal (115, 515) output and/or routed by the device (110, 510); and
obtain a value for one or more parameters of the second received signal, wherein each of the one or more parameters corresponds to a parameter of the first received signal (535); and
the step of processing the value for each of the one or more parameters to determine position information for the device comprises processing the values for the first and second received signals to determine a position of the device relative to each of the first and second sensors.

9. The processing system (140, 540) of any of claims 1 to 8, wherein:
the processing system is further configured to repeat the steps of receiving a received signal and obtaining a value for one or more parameters of the received signal for a plurality of different acoustic signals output and/or routed by the device (110, 510) from a single position; and
the step of processing the value for each of the one or more parameters to determine position information for the device comprises processing the values for each of the received signals.

10. The processing system (140, 540) of any of claims 1 to 9, wherein the processing system is further configured to:
receive information from the device (110, 510) responsive to the one or more acoustic signals output and/or routed by the device; and
determine whether a received signal corresponds to one of the one or more acoustic signals output and/or routed by the device.

11. The processing system (140, 540) of any of claims 1 to 10, wherein the processing system is further configured to output an indication of the determined position information to a display device (160, 560).

12. The processing system (140, 540) of any of claims 1 to 11, wherein:
the device (110, 510) is an ultrasound imaging device;
the step of processing the value for each of the one or more parameters to determine position information for the device comprises determining whether the device is in a desired position; and
the processing system is further configured to instruct a display device (160, 560) to display an image corresponding to imaging data received by the device in response to determining that the device is in a desired position.

13. A system (100, 500) for determining position information for a device (110, 510) within a body of a subject (120, 520), wherein the device is capable of outputting and/or routing an acoustic signal (115, 515) having a frequency of no more than 20 kHz, the system comprising:
at least one sensor (130, 530, 570) positioned, when in use, externally to the body of the subject and configured to receive an acoustic signal output and/or routed by the device; and
the processing system (140, 540) of any of claims 1 to 12.

14. A computer-implemented method (600) for determining position information for a device (110, 510) within a body of a subject (120, 520), wherein the device is capable of outputting and/or routing an acoustic signal (115, 515) having a frequency of no more than 20 kHz, the computer-implemented method comprising:
receiving, from a first sensor (130, 530) positioned externally to the body of the subject, a first received signal (135, 535) responsive to the acoustic signal output and/or routed by the device;
obtaining a value for one or more parameters of the first received signal, wherein each of the one or more parameters is a parameter of the first received signal that varies with the distance traveled by the acoustic signal; and
processing the value for each of the one or more parameters to determine position information for the device.

15. A computer program product comprising computer program code means which, when executed on a computing device have a processing system, cause the processing system to perform all of the steps of the method (600) according to claim 14.

## Patentansprüche

1. Ein Verarbeitungssystem (140, 540) zum Bestimmen von Positionsinformationen für eine Vorrichtung (110, 510) innerhalb eines Körpers eines Patienten (120, 520), wobei die Vorrichtung in der Lage ist, ein akustisches Signal (115, 515) mit einer Frequenz von nicht mehr als 20 kHz auszugeben und/oder weiterzuleiten, wobei das Verarbeitungssystem konfiguriert ist, um: von einem ersten Sensor (130, 530), der außerhalb des Körpers des Patienten positioniert ist, ein erstes empfangenes Signal (135, 535) zu empfangen, das auf das von der Vorrichtung ausgegebene und/oder weitergeleitete akustische Signal reagiert; einen Wert für einen oder mehrere Parameter des ersten empfangenen Signals zu erhalten, wobei jeder des einen oder der mehreren Parameter ein Parameter des ersten empfangenen Signals ist, der sich mit der von dem akustischen Signal zurückgelegten Entfernung ändert; und den Wert für jeden des einen oder der mehreren Parameter zu verarbeiten, um die Positionsinformationen für das Gerät zu bestimmen.

2. Verarbeitungssystem (140, 540) nach Anspruch 1, wobei die Vorrichtung (110, 510) so konfiguriert ist, dass sie während eines Ultraschallabbildungsverfahrens akustische Signale mit einer Frequenz von nicht mehr als 20 kHz aussendet.

3. Verarbeitungssystem (140, 540) nach Anspruch 1 oder 2, wobei die Vorrichtung (110, 510) eine transösophageale Echokardiographiesonde umfasst.

4. Verarbeitungssystem (140, 540) nach einem der Ansprüche 1 bis 3, wobei der erste Sensor (130, 530) bei der Verwendung auf dem Brustkorb des Patienten (120, 520) positioniert ist.

5. Verarbeitungssystem (140, 540) nach einem der Ansprüche 1 bis 4, wobei der eine oder die mehreren Parameter des ersten empfangenen Signals (135, 535) mindestens einen der folgenden umfassen: Eine Intensität des ersten empfangenen Signals, eine Amplitude des ersten empfangenen Signals, eine Laufzeit des ersten empfangenen Signals und/oder eine Frequenz des ersten empfangenen Signals.

6. Das Verarbeitungssystem (140, 540) nach einem der Ansprüche 1 bis 5, wobei: das Verarbeitungssystem weiterhin so konfiguriert ist, dass es die Schritte des Empfangens eines empfangenen Signals und des Erhaltens eines Wertes für einen oder mehrere Parameter des empfangenen Signals für eine Vielzahl von Positionen der Vorrichtung (110, 510) wiederholt; und der Schritt des Verarbeitens des Wertes für jeden des einen oder der mehreren Parameter, um Positionsinformationen für die Vorrichtung zu bestimmen, umfasst das Verarbeiten der Werte entsprechend jeder der mehreren Positionen der Vorrichtung, um eine Position der Vorrichtung zu bestimmen, die mindestens einem der folgenden Werte entspricht: Einem Maximalwert eines Parameters, einem Minimalwert eines Parameters und/oder einem Wert eines Parameters, der einem Wendepunkt entspricht.

7. Verarbeitungssystem (140, 540) nach einem der Ansprüche 1 bis 6, wobei der Schritt des Verarbeitens des Wertes für jeden des einen oder der mehreren Parameter, um Positionsinformationen für die Vorrichtung (110, 510) zu bestimmen, ferner einen Referenzwert für jeden des einen oder der mehreren Parameter verwendet, der einer Referenzposition der Vorrichtung entspricht; und die Positionsinformationen eine Position der Vorrichtung relativ zu einer Referenzposition umfassen.

8. Verarbeitungssystem (140, 540) nach einem der Ansprüche 1 bis 7, wobei: das Verarbeitungssystem außerdem so konfiguriert ist zum: empfangen, von einem zweiten Sensor (570), der sich außerhalb des Körpers des Patienten in einer anderen Position als der erste Sensor (130, 530) befindet, eines zweiten empfangenen Signals (575), das auf das gleiche akustische Signal (115, 515) anspricht, das von der Vorrichtung (110, 510) ausgegeben und/oder weitergeleitet wird; und erhalten eines Wertes für einen oder mehrere Parameter des zweiten empfangenen Signals, wobei jeder der ein oder mehreren Parameter einem Parameter des ersten empfangenen Signals (535) entspricht; und der Schritt des Verarbeitens des Wertes für jeden des einen oder der mehreren Parameter, um Positionsinformationen für das Gerät zu bestimmen, umfasst das Verarbeiten der Werte für die ersten und zweiten empfangenen Signale, um eine Position des Geräts relativ zu jedem der ersten und zweiten Sensoren zu bestimmen.

9. Verarbeitungssystem (140, 540) nach einem der Ansprüche 1 bis 8, wobei: das Verarbeitungssystem ferner so konfiguriert ist, dass es die Schritte des Empfangens eines empfangenen Signals und des Erhaltens eines Wertes für einen oder mehrere Parameter des empfangenen Signals für eine Vielzahl von verschiedenen akustischen Signalen, die von der Vorrichtung (110, 510) von einer einzigen Position aus ausgegeben und/oder geleitet werden, wiederholt; und der Schritt der Verarbeitung des Wertes für jeden des einen oder der mehreren Parameter zur Bestimmung der Positionsinformationen für das Gerät die Verarbeitung der Werte für jedes der empfangenen Signale umfasst.

10. Verarbeitungssystem (140, 540) nach einem der Ansprüche 1 bis 9, wobei das Verarbeitungssystem ferner so konfiguriert ist zum: empfangen von Informationen von der Vorrichtung (110, 510) in Reaktion auf die ein oder mehreren akustischen Signale, die von der Vorrichtung ausgegeben und/oder weitergeleitet werden; und feststellen, ob ein empfangenes Signal einem des einen oder der mehreren akustischen Signale entspricht, die von dem Gerät ausgegeben und/oder weitergeleitet werden.

11. Verarbeitungssystem (140, 540) nach einem der Ansprüche 1 bis 10, wobei das Verarbeitungssystem ferner so konfiguriert ist, dass es eine Anzeige der ermittelten Positionsinformationen an eine Anzeigevorrichtung (160, 560) ausgibt.

12. Verarbeitungssystem (140, 540) nach einem der Ansprüche 1 bis 11, wobei; das Gerät (110, 510) ein Ultraschall-Bildgebungsgerät ist; der Schritt der Verarbeitung des Wertes für jeden der ein oder mehreren Parameter, um die Positionsinformation für die Vorrichtung zu bestimmen, umfasst die Bestimmung, ob sich die Vorrichtung in einer gewünschten Position befindet; und das Verarbeitungssystem ist außerdem so konfiguriert, dass es eine Anzeigevorrichtung (160, 560) anweist, ein Bild anzuzeigen, das den von der Vorrichtung empfangenen Bilddaten entspricht, wenn festgestellt wird, dass sich die Vorrichtung in einer gewünschten Position befindet.

13. System (100, 500) zur Bestimmung von Positionsinformationen für eine Vorrichtung (110, 510) innerhalb eines Körpers eines Patienten (120, 520), wobei die Vorrichtung in der Lage ist, ein akustisches Signal (115, 515) mit einer Frequenz von nicht mehr als 20 kHz auszugeben und/oder weiterzuleiten, wobei das System umfasst: mindestens einen Sensor (130, 530, 570), der im Gebrauch außerhalb des Körpers des Patienten angeordnet und so konfiguriert ist, dass er ein von der Vorrichtung ausgegebenes und/oder weitergeleitetes akustisches Signal empfängt; und das Verarbeitungssystem (140, 540) nach einem der Ansprüche 1 bis 12.

14. Durch Computer verwirklichtes Verfahren (600) zum Bestimmen von Positionsinformationen für eine Vorrichtung (110, 510) innerhalb eines Körpers eines Patienten (120, 520), wobei die Vorrichtung in der Lage ist, ein akustisches Signal (115, 515) mit einer Frequenz von nicht mehr als 20 kHz auszugeben und/oder weiterzuleiten, wobei das durch Computer verwirklichte Verfahren umfasst: empfangen eines ersten empfangenen Signals (135, 535) von einem ersten Sensor (130, 530), der außerhalb des Körpers des Patienten positioniert ist, als Reaktion auf das von der Vorrichtung ausgegebene und/oder weitergeleitete akustische Signal; erhalten eines Wertes für einen oder mehrere Parameter des ersten empfangenen Signals, wobei jeder des einen oder der mehreren Parameter ein Parameter des ersten empfangenen Signals ist, der sich mit der von dem akustischen Signal durchlaufenen Entfernung ändert; und Verarbeitung des Wertes für jeden der ein oder mehreren Parameter zur Bestimmung der Positionsinformationen für das Gerät.

15. Computerprogrammprodukt, das Computerprogrammcodemittel umfasst, die, wenn sie auf einem Computergerät mit einem Verarbeitungssystem ausgeführt werden, das Verarbeitungssystem veranlassen, alle Schritte des Verfahrens (600) nach Anspruch 14 auszuführen.

## Revendications

1. Système de traitement (140, 540) pour déterminer les informations de position d'un dispositif (110, 510) à l'intérieur du corps d'un sujet (120, 520), dans lequel le dispositif est capable d'émettre et/ou d'acheminer un signal acoustique (115, 515) ayant une fréquence ne dépassant pas 20 kHz, le système de traitement étant configuré pour: recevoir, d'un premier capteur (130, 530) placé à l'extérieur du corps du sujet, un premier signal reçu (135, 535) répondant au signal acoustique émis et/ou acheminé par le dispositif; obtenir une valeur pour un ou plusieurs paramètres du premier signal reçu, dans lequel chacun des un ou plusieurs paramètres est un paramètre du premier signal reçu qui varie en fonction de la distance parcourue par le signal acoustique; et traiter la valeur de chacun des paramètres afin de déterminer la position du dispositif.

2. Système de traitement (140, 540) selon la revendication 1, dans lequel le dispositif (110, 510) est configuré pour émettre des signaux acoustiques ayant une fréquence ne dépassant pas 20 kHz au cours d'un processus d'imagerie par ultrasons.

3. Système de traitement (140, 540) selon la revendication 1 ou 2, dans lequel le dispositif (110, 510) comprend une sonde d'échocardiographie transœsophagienne.

4. Système de traitement (140, 540) selon l'une des revendications 1 à 3, dans lequel le premier capteur (130, 530) est, en cours d'utilisation, placé sur le thorax du sujet (120, 520).

5. Système de traitement (140, 540) selon l'une des revendications 1 à 4, dans lequel le ou les paramètres du premier signal reçu (135, 535) comprennent au moins l'un des éléments suivants : une intensité du premier signal reçu, une amplitude du premier signal reçu, un temps de vol du premier signal reçu et/ou une fréquence du premier signal reçu.

6. Système de traitement (140, 540) selon l'une des revendications 1 à 5, dans lequel: le système de traitement est en outre configuré pour répéter les étapes de réception d'un signal reçu et d'obtention d'une valeur pour un ou plusieurs paramètres du signal reçu pour une pluralité de positions du dispositif (110, 510); et l'étape de traitement de la valeur de chacun des un ou plusieurs paramètres afin de déterminer les informations relatives à la position du dispositif consiste à traiter les valeurs correspondant à chacune de la pluralité de positions du dispositif afin de déterminer une position du dispositif correspondant à au moins l'une des valeurs suivantes: une valeur maximale d'un paramètre, une valeur minimale d'un paramètre et/ou une valeur d'un paramètre correspondant à un point d'inflexion.

7. Système de traitement (140, 540) selon l'une des revendications 1 à 6, dans lequel: l'étape de traitement de la valeur de chacun des un ou plusieurs paramètres pour déterminer l'information de position pour le dispositif (110, 510) utilise en outre une valeur de référence pour chacun des un ou plusieurs paramètres correspondant à une position de référence du dispositif; et l'information de position comprend une position du dispositif par rapport à une position de référence.

8. Système de traitement (140, 540) selon l'une des revendications 1 à 7, dans lequel: le système de traitement est en outre configuré pour: recevoir, d'un deuxième capteur (570) positionné à l'extérieur du corps du sujet dans une position différente de celle du premier capteur (130, 530), un deuxième signal reçu (575) répondant au même signal acoustique (115, 515) émis et/ou acheminé par le dispositif (110, 510); et obtenir une valeur pour un ou plusieurs paramètres du deuxième signal reçu, dans lequel chacun des paramètres correspond à un paramètre du premier signal reçu (535); et l'étape consistant à traiter la valeur de chacun des un ou plusieurs paramètres pour déterminer les informations relatives à la position du dispositif consiste à traiter les valeurs des premier et deuxième signaux reçus pour déterminer la position du dispositif par rapport à chacun des premier et deuxième capteurs.

9. Système de traitement (140, 540) selon l'une des revendications 1 à 8, dans lequel: le système de traitement est en outre configuré pour répéter les étapes de réception d'un signal reçu et d'obtention d'une valeur pour un ou plusieurs paramètres du signal reçu pour une pluralité de signaux acoustiques différents émis et/ou acheminés par le dispositif (110, 510) à partir d'une position unique; et l'étape consistant à traiter la valeur de chacun des un ou plusieurs paramètres afin de déterminer les informations relatives à la position du dispositif consiste à traiter les valeurs de chacun des signaux reçus.

10. Système de traitement (140, 540) selon l'une des revendications 1 à 9, dans lequel le système de traitement est en outre configuré pour: recevoir des informations du dispositif (110, 510) en réponse à un ou plusieurs signaux acoustiques émis et/ou acheminés par le dispositif; et déterminer si un signal reçu correspond à l'un des signaux acoustiques émis et/ou acheminés par le dispositif.

11. Système de traitement (140, 540) selon l'une des revendications 1 à 10, dans lequel le système de traitement est en outre configuré pour fournir une indication des informations de position déterminées à un dispositif d'affichage (160, 560).

12. Système de traitement (140, 540) selon l'une des revendications 1 à 11, dans lequel: le dispositif (110, 510) est un dispositif d'imagerie par ultrasons;
l'étape de traitement de la valeur de chacun des un ou plusieurs paramètres pour déterminer les informations de position pour le dispositif consiste à déterminer si le dispositif est dans une position souhaitée; et l'information sur la position du dispositif consiste à déterminer si le dispositif est dans une position souhaitée; et le système de traitement est en outre configuré pour ordonner à un dispositif d'affichage (160, 560) d'afficher une image correspondant aux données d'imagerie reçues par le dispositif en réponse à la détermination que le dispositif est dans une position souhaitée.

13. Système (100, 500) permettant de déterminer des informations sur la position d'un dispositif (110, 510) à l'intérieur du corps d'un sujet (120, 520), dans lequel le dispositif est capable d'émettre et/ou d'acheminer un signal acoustique (115, 515) ayant une fréquence ne dépassant pas 20 kHz, le système comprenant: au moins un capteur (130, 530, 570) positionné, lorsqu'il est utilisé, à l'extérieur du corps du sujet et configuré pour recevoir un signal acoustique émis et/ou acheminé par le dispositif; et le système de traitement (140, 540) selon l'une quelconque des revendications 1 à 12.

14. Méthode (600) mise en œuvre par ordinateur pour déterminer les informations de position d'un dispositif (110, 510) dans le corps d'un sujet (120, 520), dans laquelle le dispositif est capable d'émettre et/ou d'acheminer un signal acoustique (115, 515) ayant une fréquence ne dépassant pas 20 kHz, la méthode mise en œuvre par ordinateur comprenant les étapes consistant à: recevoir, d'un premier capteur (130, 530) placé à l'extérieur du corps du sujet, un premier signal reçu (135, 535) en réponse au signal acoustique émis et/ou acheminé par le dispositif; obtenir une valeur pour un ou plusieurs paramètres du premier signal reçu, dans lequel chacun des paramètres est un paramètre du premier signal reçu qui varie en fonction de la distance parcourue par le signal acoustique; et traiter la valeur de chacun des paramètres pour déterminer la position du dispositif.

15. Produit de programme informatique comprenant des moyens de code de programme d'ordinateur qui, lorsqu'ils sont exécutés sur un dispositif informatique doté d'un système de traitement, amènent le système de traitement à exécuter toutes les étapes de la méthode (600) selon la revendication 14.
